# EUROPEAN PATENT SPECIFICATION

(11) **EP 2 674 111 B1**
(45) Date of publication and mention of the grant of the patent: **08.03.2017**
(21) Application number: 13171803.3
(22) Date of filing: 13.06.2013
(51) Int. Cl.: A61B 17/072, A61B 17/29

(54) **Sliding anvil/retracting cartridge reload**
Gleitende Amboss-/Einfahrkartuschen-Neuladung
Rechargement de cartouche de rétraction / enclume coulissante

(30) Priority: 14.06.2012 US 201261659567 P; 05.04.2013 US 201313857202
(43) Date of publication of application: 18.12.2013
(73) Proprietor: Covidien LP, Mansfield, MA 02048 (US)
(72) Inventor: Krehel, Gregg, Newtown, Connecticut 06470 (US); Cabrera, Ramiro, Cheshire, Connecticut 06410 (US); Whitfield, Kenneth, North Haven, Connecticut 06473 (US); Racenet, Danyel, Killingworth, Connecticut 06419 (US)
(74) Representative: Maschio, Antonio

(56) References cited:
- EP-A1- 2 165 654
- EP-A1- 2 604 193
- EP-A2- 0 769 273
- US-A- 5 403 326
- US-A- 5 413 267
- US-A1- 2011 272 448
- US-A1- 2012 012 636
- US-A1- 2012 143 218

## Description

### BACKGROUND

### 1. Technical Field

The present disclosure relates to a surgical stapling device and, more particularly, to an endoscopic surgical stapling device configured to operate a tool assembly with an extendable tip element for manipulating tissue that is usable in thoracic procedures.

### 2. Background of Related Art

Surgical devices wherein tissue is first grasped or clamped between opposing jaw structures and then joined by surgical fasteners are well known in the art. The fasteners are typically in the form of surgical staples, but two-part polymeric fasteners can also be utilized.

Instruments for this purpose can include two elongated members which are respectively used to capture or clamp tissue. Typically, one of the members carries a staple cartridge which houses a plurality of staples arranged, for example, in at least two lateral rows while the other member has an anvil that defines a surface for forming the staple legs as the staples are driven from the staple cartridge. Generally, the stapling operation is effected by sleds that travel longitudinally through the staple cartridge, with the sleds acting upon staple pushers to sequentially eject the staples from the staple cartridge. A knife can travel between the staple rows to longitudinally cut the stapled tissue between the rows of staples. Such staplers are disclosed in U.S. Patent Nos. 6,250,532 and 6,241,139.

In minimally invasive thoracic procedures, surgery is performed through small incisions or through small diameter cannulas or seal anchors inserted through small entrance wounds in the skin. During minimally invasive thoracic procedures in particular, surgery is performed through the natural intercostal space between adjacent ribs. Due to the limited degree of motion of an instrument when it is positioned between the ribs, it may be quite difficult for a surgeon to manipulate the tool assembly of the instrument around body tissue extraneous to the procedure to access and/or clamp the tissue site. Instruments having rotatable endoscopic body portions and rotatable and/or articulatable tool assemblies have been developed to overcome this problem and are commercially available. Although these instruments provide significant improvements in the endoscopic tool art, further improvements that may decrease the time required for surgical procedures by allowing surgeons to more easily access tissue sites are desired. Accordingly, a continuing need exists for a minimally invasive surgical device having a tool assembly that can quickly and easily manipulate tissue.

US2012/0143218A describes a surgical stapler having an extendable dissection tip according to the preamble of claim 1.

### SUMMARY

The present invention provides a surgical device comprising: a handle assembly; an elongated member extending distally from the handle assembly, the elongated member defining a longitudinal axis; a tool assembly mounted to a distal end of the elongated member, the tool assembly having a cartridge assembly having a plurality of staples supported therein and an anvil assembly, at least one of the anvil assembly and the cartridge assembly being pivotably movable in relation to each other between open and closed positions, wherein at least one of the cartridge assembly and the anvil assembly includes a tongue configured to transition said at least one of the cartridge assembly and the anvil assembly between an extended state and a retracted state, characterized in that the tongue is configured to transition out from and into said at least one of the cartridge assembly and the anvil assembly, and the surgical device further comprises a transitioning mechanism configured to transition at least one of the anvil assembly and the cartridge assembly between the extended and retracted states wherein the transitioning mechanism includes a rod for transitioning the tongue.

The transitioning mechanism may pneumatically transition the tongue. The tongue may have an arcuate configuration.

In some embodiments, the tongue is composed of shape memory material and returns to a shape memorized position in the extended state.

In some embodiments, at least one of the cartridge assembly and the anvil assembly distal to an engagement plane may transition between the expanded state and the contracted state. At least one of the cartridge assembly and the anvil assembly proximal to an engagement plane may remain stationary as at least one of the cartridge assembly and the anvil assembly distal to an engagement plane transitions between the expanded state and the contracted state.

In some embodiments, at least one of the cartridge assembly and the anvil assembly proximal to an engagement plane may transition between the expanded state and the contracted state. At least one of the cartridge assembly and the anvil assembly distal to an engagement plane may remain stationary as at least one of the cartridge assembly and the anvil assembly proximal to an engagement plane transitions between the expanded state and the contracted state.

The cartridge assembly and the anvil assembly may move between the open and closed positions in response to actuation of a movable handle of the handle assembly. The cartridge assembly may fire the plurality of staples in response to actuation of a movable handle of the handle assembly. The surgical device may include a mode selection mechanism configured to alternate the surgical device between a first mode of operation and a second mode of operation. The mode selection mechanism may include a switch for selecting between the first mode of operation, in which the cartridge assembly and anvil assembly are movable back and forth between the open and closed positions, and the second mode of operation, in which the cartridge assembly and anvil assembly are locked in the closed position for clamping tissue and firing the plurality of staples.

The surgical device of the invention may be used in a method of stapling tissue having the steps of inserting a surgical stapling device including an end effector having an extendable tongue through an incision in tissue, extending the extendable tongue, manipulating tissue with the extendable tongue, grasping tissue within the end effector, switching the surgical stapling device from a grasping mode to a firing mode, and firing staples from the end effector through tissue. The method may further include the step of pulling a retraction handle of the surgical stapling device to release tissue from within the end effector and return the surgical stapling device from the firing mode to the grasping mode. The extendable tongue is movable out from and into an anvil assembly or cartridge assembly of the end effector. The extendable tongue may be curved.

### BRIEF DESCRIPTION OF THE DRAWINGS

Embodiments of the presently disclosed surgical stapling device are disclosed herein with reference to the drawings wherein:
FIG. 1A is a side perspective view of a surgical stapling device according to the present disclosure;
FIG. 1B is a top view illustrating the surgical stapling device of FIG. 1 A;
FIG. 1C is a side view illustrating the surgical stapling device of FIG. 1A;
FIG. 2A is an exploded view of a handle assembly of the surgical stapling device of FIG. 1 A;
FIG. 2B is a side, perspective view of an actuation bar of the handle assembly of FIG.2A;
FIG. 2C is a side, cross-sectional view of the actuation bar of FIG. 2B;
FIG. 3A is a side, cross-section view of the handle assembly of FIG. 2A with a movable handle thereof in an first position;
FIG. 3B is a side, cross-sectional view of a pawl assembly of the handle assembly of FIG. 2A;
FIG. 3C is a side, cross-sectional view of the pawl assembly of FIG. 3B with the movable handle in a second position;
FIG. 3D is a side, perspective view of the handle mechanism of FIG. 2A being transitioned from a grasping mode to a firing mode;
FIG. 3E is a side, cross-sectional view of the pawl assembly of FIG. 3B in the firing mode while the movable handle is in the first position;
FIG. 3F is a side, perspective view of the pawl assembly of FIG. 3B;
FIG. 3G is a side perspective view of the pawl assembly of FIG. 3F in the firing mode as the movable handle is returning to the second position;
FIG. 3H is a side, cross-sectional view of the pawl assembly of FIG. 3G;
FIG. 3I is a side, cross-sectional view of the pawl assembly of FIG. 3H as the movable handle is pulled to the first position while in firing mode;
FIG. 4 is a side perspective view illustrating an end effector of the surgical stapling device of FIG. 1A in an open position with a tongue extended therefrom;
FIG. 5 is a front cross-sectional view of the end effector of FIG. 4;
FIG. 6A is a side cross-sectional view of a tool assembly of the end effector of FIG. 4 in a closed position with the tongue retracted therein;
FIG. 6B is a side cross-sectional view of the tool assembly of FIG. 6A in an open position with the tongue extended therefrom;
FIG. 7 is a side cross-sectional view of the tool assembly of FIG. 6A illustrating distal translation of a drive member and a sled;
FIG. 8 is a side cross-sectional view of the tool assembly of FIG. 6A illustrating firing of a plurality of staples;
FIG. 9A is a side, cross-sectional view of the surgical stapling device of FIG. 1A prior to entry into an opening in thoracic tissue in a minimally invasive thoracic procedure;
FIG. 9B is a side, cross-sectional view of the surgical stapling device of FIG. 1A inserted through the opening in thoracic tissue and manipulating a vessel therein;
FIG. 9C is a side, cross-sectional view of the surgical stapling device of FIG. 1A stapling the vessel;
FIG. 10 is a side perspective view of an anvil assembly of a surgical stapling device according to an embodiment of the present disclosure having a pneumatically controlled tongue;
FIG. 11 is a side view of a tool assembly of a surgical stapling device not in accordance with the present invention having a retractable cartridge assembly; and
FIG. 12 is a side view of an end effector of a surgical stapling device not in accordance with the present invention having an extendable body portion, an LED tip, and a tube.

### DETAILED DESCRIPTION OF THE EMBODIMENTS

Embodiments of the presently disclosed surgical stapling device will now be described in detail with reference to the drawings in which like reference numerals designate identical or corresponding element in each of the several views.

Throughout this description, the term "proximal" will refer to the portion of the device closer to the operator and the term "distal" will refer to the portion of the device further from the operator.

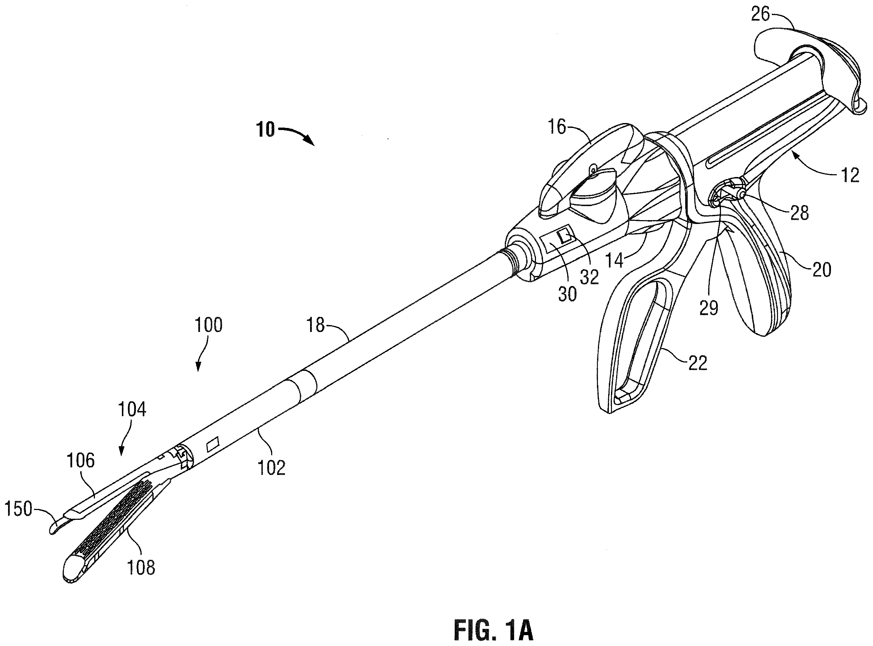

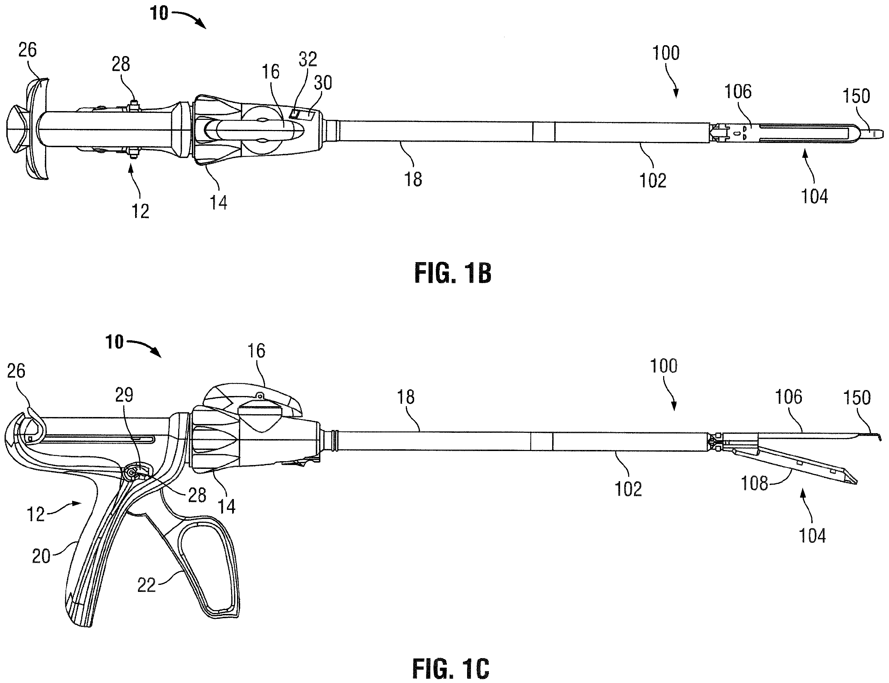

FIGS. 1A-1C illustrate one embodiment of the presently disclosed surgical stapling device shown generally as 10. Surgical stapling device 10 includes a handle assembly 12, a rotation knob 14, an articulation lever 16, an elongated shaft 18, and an end effector 100. Handle assembly 12 includes a stationary handle 20, a movable handle 22, and retraction handle 26. Elongated shaft 18 defines a longitudinal axis of surgical stapling device 10. An actuator button 28 extends transversely through and projects outwardly from button paths 29 on opposite sides of handle assembly 12. Actuator button 28 is slidable along button paths 29. A translation switch 32 is slidable within a switch depression 30 on handle assembly 12. Surgical stapling device 10 is transitionable between a grasping mode, in which end effector 100 may grasp and release tissue, and a firing mode, in which end effector 100 fires staples through tissue.

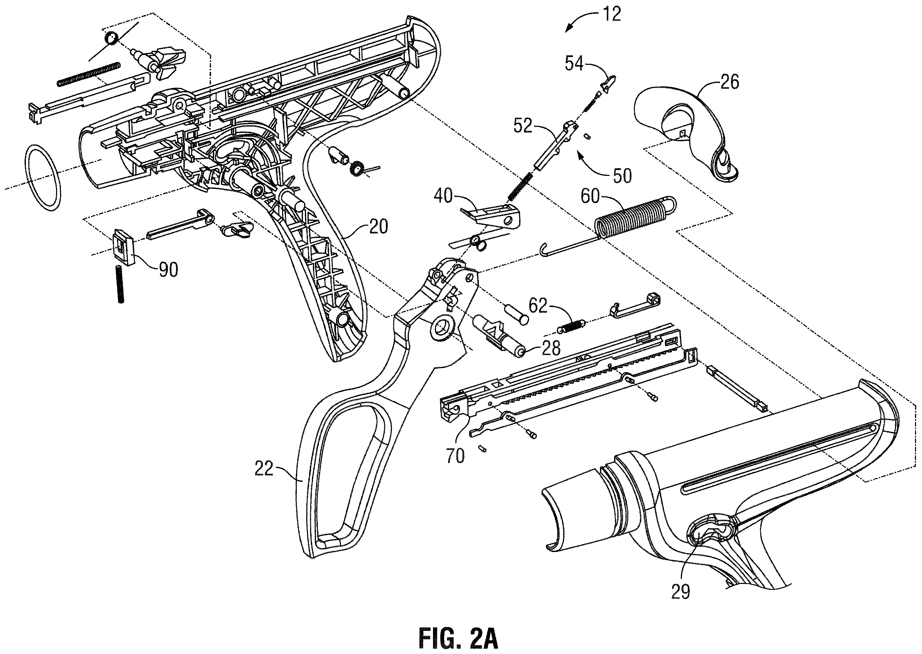

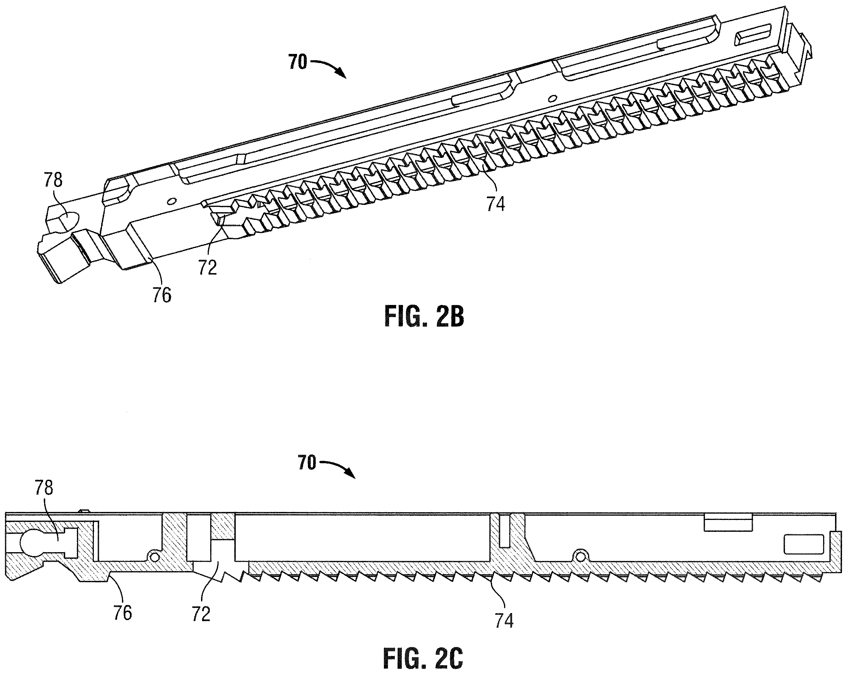

Movable handle 22 pivotably transitions between a first position and a second position. Movable handle 22 is biased by a handle spring 60 (FIG. 2A) toward the second position, in which movable handle 22 is at a farthest distance from stationary handle 20. Pulling movable handle 22 toward stationary handle 20 transitions movable handle 22 to the first position, in which movable handle 22 is at a closest distance to stationary handle 20. Releasing movable handle 22 allows biasing forces within handle assembly 12 to transition movable handle 22 back to the second position.

End effector 100 includes a body portion 102 and a tool assembly 104 pivotably attached to body portion 102. Rotation knob 14 may be rotated to actuate rotation of end effector 100 about the longitudinal axis. Rotation of rotation knob 14 may rotate elongated shaft 18 and end effector 100. Articulation lever 16 may be turned to actuate articulation of tool assembly 104, which moves tool assembly 104 off axis relative to the longitudinal axis. In some embodiments, end effector 100 may be a disposable loading unit (DLU) that is releasably attached to elongated shaft 18.

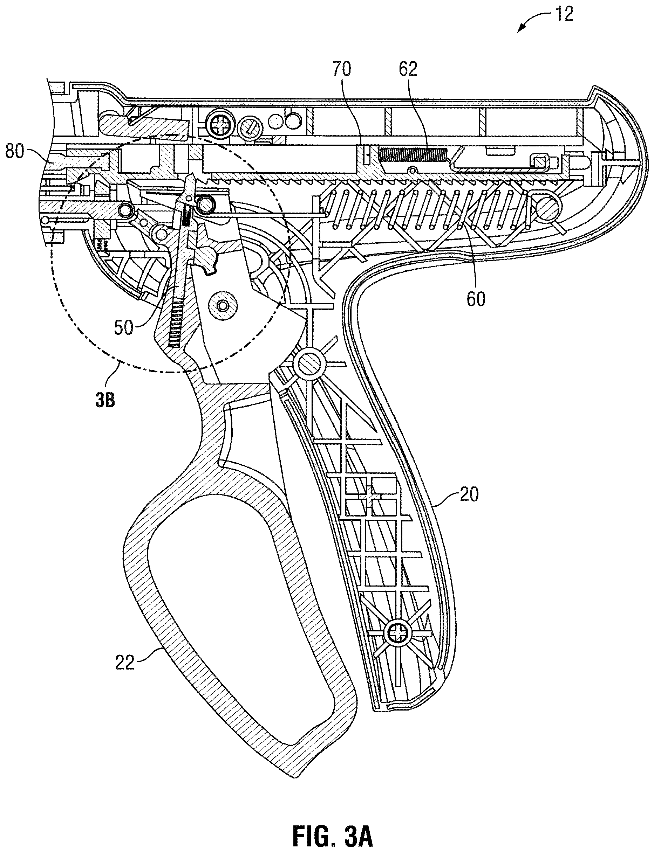

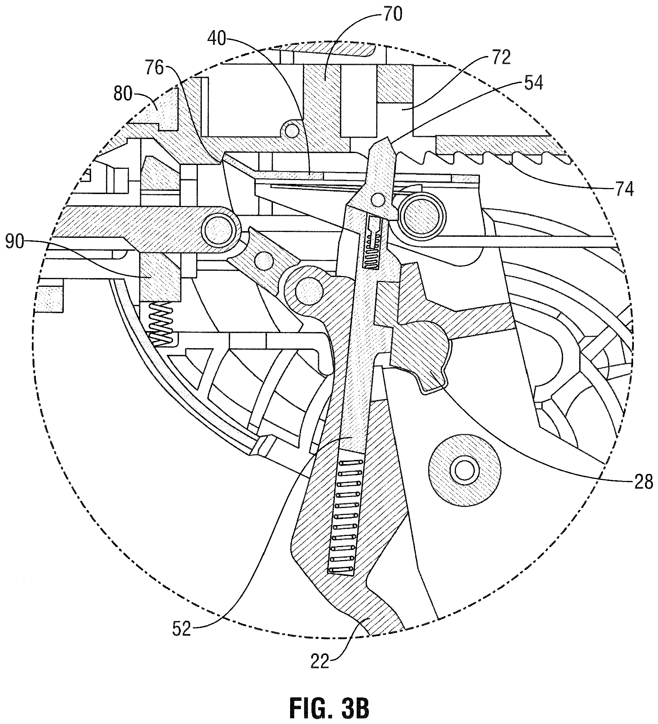

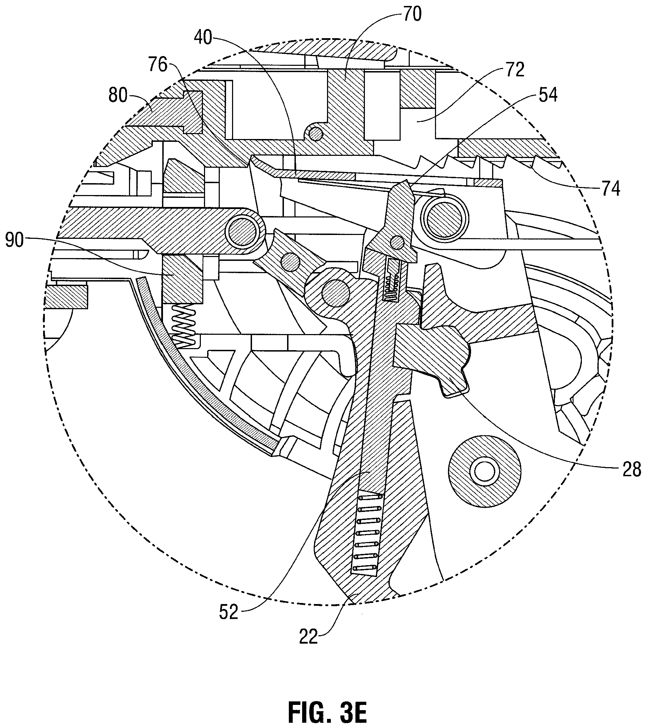

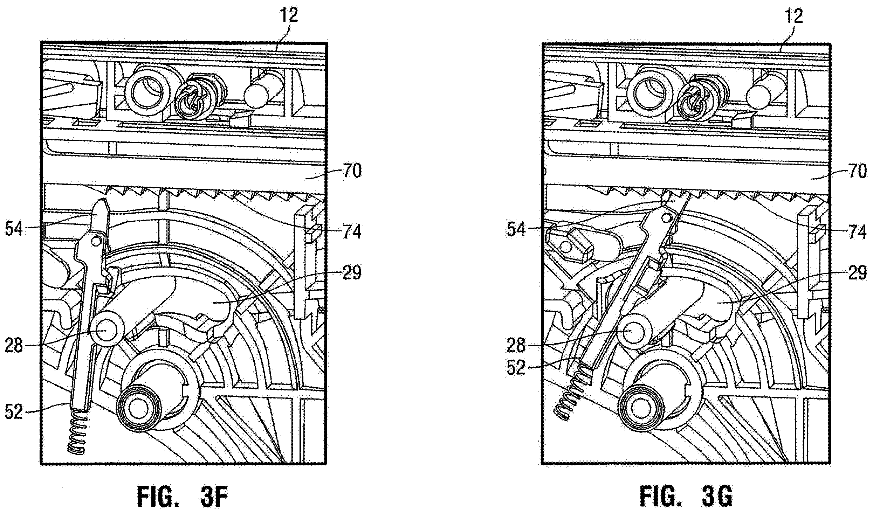

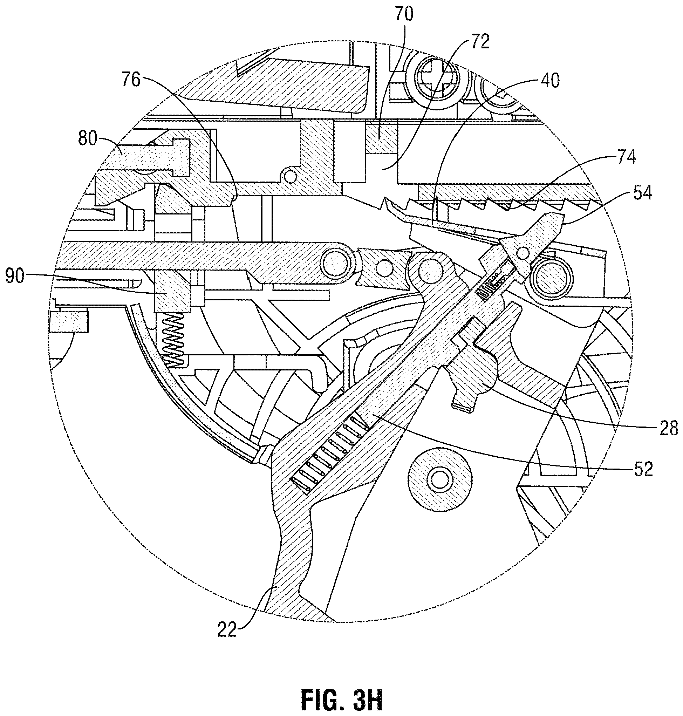

As seen in FIG. 2A, handle assembly 12 includes a pawl assembly 50. Pawl assembly 50 includes a pawl arm 52 and a grasping pawl 54. Handle assembly 12 further includes a driving pawl 40 and a vertical pawl 90. Handle assembly 12 further includes handle spring 60 for biasing movable handle 22 toward the second position and an actuator spring 62 for biasing an actuator bar 70 proximally. Actuator bar 70 translates an actuation rod 80 (FIG. 3A) for actuating grasping and firing of tool assembly 104. Pawl arm 52 is operably connected to actuator button 28 such that a distal sliding of actuator button 28 retracts pawl assembly 50 as will be discussed in greater detail hereinbelow.

As seen in FIGS. 2B-2C, actuator bar 70 has a pawl gap 72, teeth 74, an abutment 76, and a rod recess 78. Rod recess 78 is configured and dimensioned to receive a proximal end of actuation rod 80 therein. Pawl gap 72 is configured and dimensioned to removably receive pawl 54 therein when surgical stapling device 10 is in the grasping mode. Abutment 76 is configured to engage driving pawl 42 to drive actuation bar 70 distally in the grasping mode. Teeth 74 are configured to engage driving pawl 42 to drive actuation bar 70 distally in the firing mode.

FIGS. 3A-3I illustrate pawl assembly 50 as surgical stapling device 10 is transitioned from the grasping mode to the firing mode. As seen in FIGS. 3A-3B, surgical stapling device 10 begins in the grasping mode. Pulling movable handle 22 from the second position to the first position urges driving pawl 40 distally. Driving pawl 40 contacts abutment 76 to drive actuator bar 70 distally. Distal translation of actuator bar 70 distally translates actuation rod 80, thereby causing end effector 100 to grasp tissue, as will be explained in greater detail hereinbelow. Grasping pawl 54 remains in pawl gap 72 in the grasping mode.

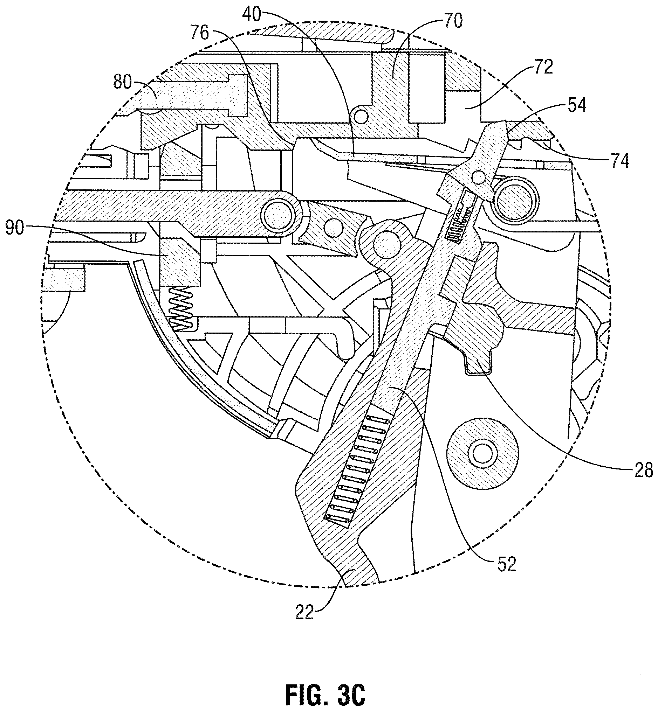

FIG. 3C illustrates pawl assembly 50 as movable handle 22 is released back to the second position. Driving pawl 40 retreats proximally from abutment 76 and thus no longer urges actuator bar 70 distally. Actuator spring 62 translates actuator bar 70 proximally, which in turn pulls actuation rod 80 proximally and causes end effector 100 to release its grasp on tissue.

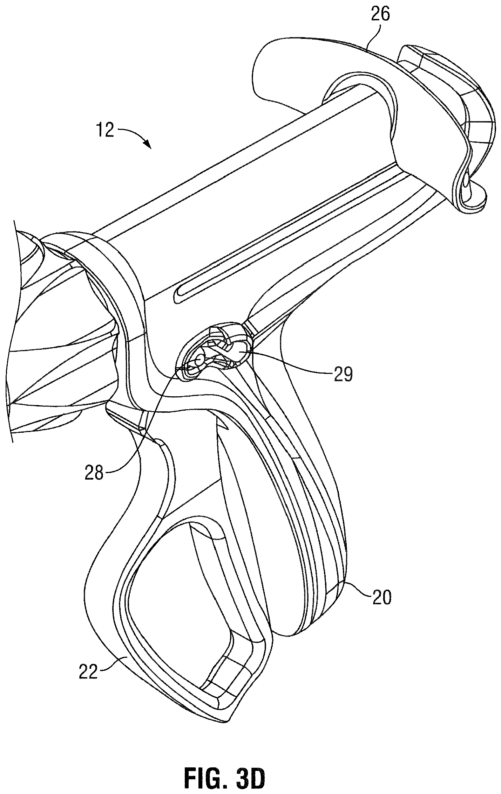

As seen in FIGS. 3D-3F, when surgical stapling device 10 is in the grasping mode and movable handle 22 is moved to the first position, actuator button 28 can be slid distally to transition surgical stapling device 10 from the grasping mode to the firing mode. Sliding actuator button 28 distally retracts grasping pawl 54 from pawl gap 72.

As seen in FIGS. 3G-3H, when movable handle 22 is released for the first time after surgical stapling device 10 is transitioned to the firing mode, pawl arm 52 rotates such that grasping pawl 54, which is no longer within pawl gap 72 and thus not inhibited from moving proximally, is moved to a position proximal to pawl gap 72. As pawl arm 52 rotates, pawl arm 52 slides actuator button 28 proximally, thereby returning pawl assembly 50 from the retracted position. Driving pawl 40 now engages teeth 74.

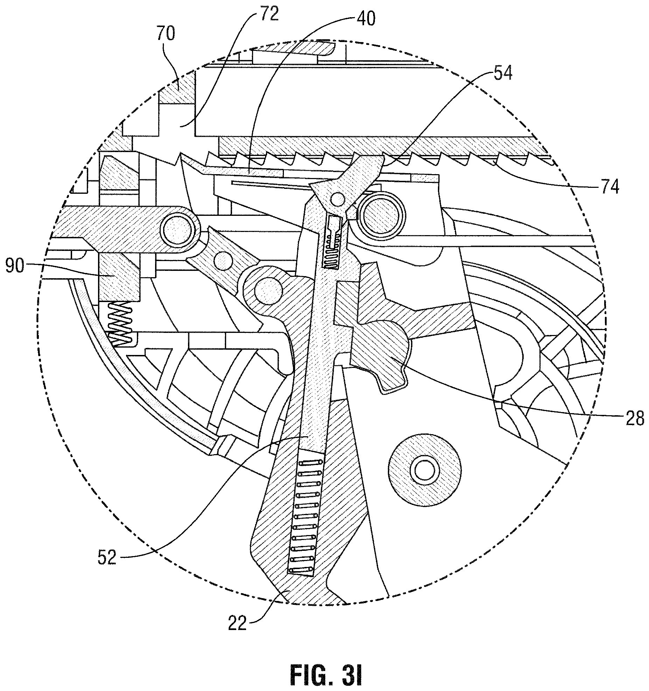

As seen in FIG. 3I, an additional pulling of movable handle 22 to the first position drives actuator bar 70 distally. Driving pawl 40 drives actuator bar 70 distally when movable handle 22 is pulled to the first position. Vertical pawl 90 engages actuator bar 70 to inhibit actuator bar 70 from being pulled proximally by actuator spring 62 as movable handle 22 is released to the second position. Grasping pawl 54 and driving pawl 40 slide proximally along teeth 74 as movable handle 22 is released to the second position. Thus, additional pulling and releasing of movable handle 22 incrementally drives actuator bar 70 distally to fire staples. When firing is complete, retraction handle 26 is pulled proximally, which removes actuator bar from contact with driving pawl 40 and grasping pawl 54 and pulls actuator bar 70 proximally. Surgical stapling device 10 is thus returned to the grasping mode.

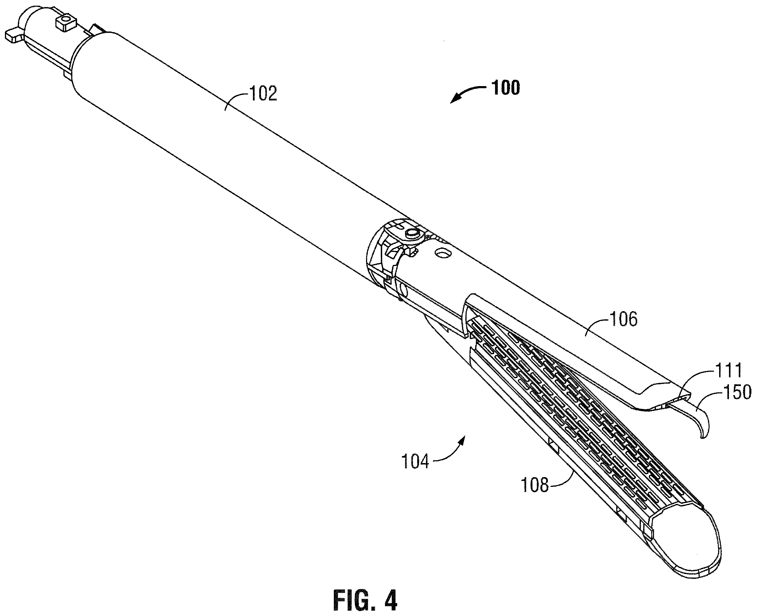

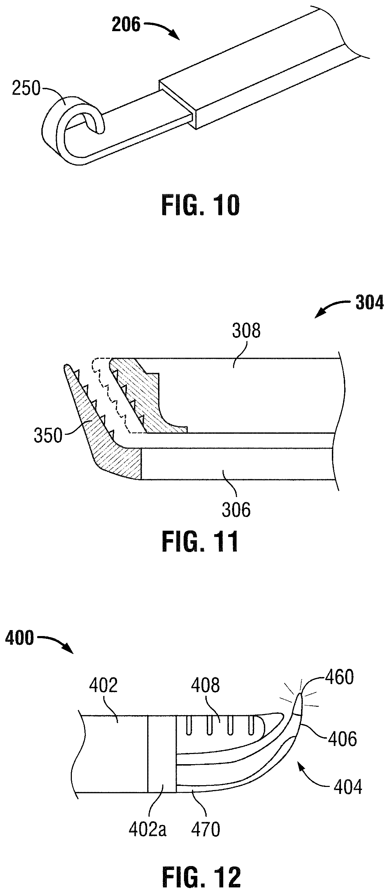

As seen in FIG. 4, tool assembly 104 includes an anvil assembly 106 and a cartridge assembly 108. Anvil assembly 106 and cartridge assembly 108 are movable back and forth between an open position and a closed position when surgical stapling device 10 is in a grasping mode. Anvil assembly 106 and cartridge assembly 108 are pivotably movable with respect to each other. In one embodiment, anvil assembly 106 and cartridge assembly 108 are also translatably movable with respect to each other (FIG. 11). In the open position, anvil assembly 106 and cartridge assembly 108 as spaced sufficiently far apart to receive a tissue therebetween. In the closed position, anvil assembly 106 and cartridge assembly 108 are spaced sufficiently close together to grasp tissue therebetween, i.e., close cooperative alignment. Anvil assembly 106 and cartridge assembly 108 may be substantially parallel in the closed position.

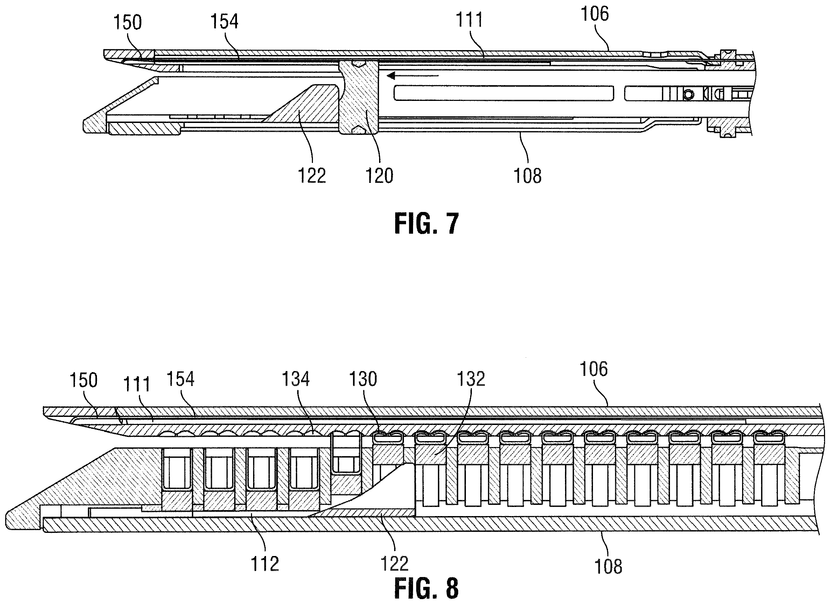

In the grasping mode, anvil assembly 106 and cartridge assembly 108 move to the closed position upon a pulling of movable handle 22 to the first position. Anvil assembly 106 and cartridge assembly 108 return to the open position as movable handle 22 is released to the second position. When tool assembly 104 is in the closed position, surgical stapling device 10 may be transitioned from the grasping mode to a firing mode by sliding actuator button 28 distally. In the firing mode, anvil assembly 106 and cartridge assembly 108 remain in the closed position as movable handle 22 is moved away from stationary handle 20. Pulling movable handle 22 to the first position once more fires staples 130 (FIG. 8) from cartridge assembly 108. Compression of staples 130 between cartridge assembly 108 and anvil assembly 106 deforms staples 130, thereby stapling tissue between cartridge assembly 108 and anvil assembly 106. Surgical stapling device 10 may be returned from the firing mode to the grasping mode by pulling retraction handles 26 proximally. Such a stapler is disclosed in US-A-20110272448.

Anvil assembly 106 includes a translatable tip 150 for selectively manipulating tissue in a surgical site. In some embodiments, cartridge assembly 108 rather than anvil assembly 106 includes a translatable tip. Embodiments are also conceived in which both anvil assembly 106 and cartridge assembly 108 include translatable tips. Translatable tip 150 is retractable within an anvil channel 111 within anvil assembly 106 upon a sliding of translation switch 32 proximally. Translatable tip 150 is extendable from anvil channel 111 upon a sliding of translation switch 32 distally. Extension and retraction of translatable tip 150 may be partial. Alternatively, translatable tip 150 may be transitioned only between a completely extended state and a completely retracted state.

Translatable tip or tongue 150 may have a generally curved configuration. Translatable tip 150 may be composed of a resiliently deformable material, such as a memory metal, such that translatable tip 150 has a substantially arcuate configuration when extended out of anvil channel 111 and a substantially linear configuration when retracted into anvil channel 111. Translatable tip 150 may have a soft durometer to assist in transitioning between the retracted and extended states. Translatable tip 150 may have any suitable curvature, for example, translatable tip 150 may curve toward cartridge assembly 108 or away from cartridge assembly 108. Embodiments are conceived in which translatable tip 150 may be rotated to curve in any desirable direction. Embodiments are also conceived in which translatable tip 150 has adjustable curvature.

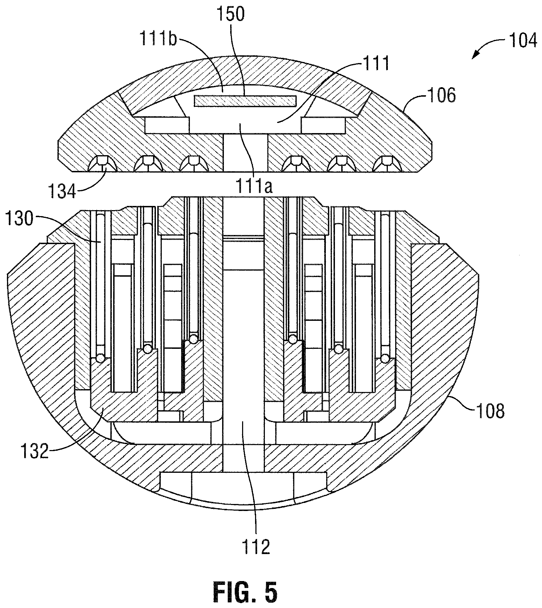

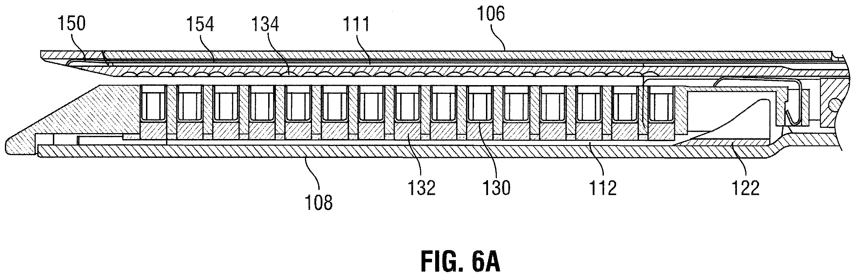

As seen in FIG. 5, anvil assembly 106 includes anvil channel 111 and cartridge assembly includes a cartridge channel 112. Channels 111, 112 are configured for passage of a drive member 120 (FIG. 7) therethrough. A proximal end of drive member 120 is operably connected to actuation rod 80 such that translation of actuation rod 80 correspondingly translates drive member 120. Anvil channel 111 may be divided into a first channel 111 a configured for passage of drive member 120 therethrough and a second channel 111b for passage of translatable tip 150 therethrough. Cartridge assembly 108 includes a plurality of staples 130 and a plurality of pushers 132 therein. Pushers 132 may be translated by a sled 122 (FIG. 6A) toward anvil cartridge 106 to push the plurality of staples 130 into contact with a corresponding plurality of anvil pockets 134 in anvil cartridge 106. Pressure between staples 130 and anvil pockets 134 deforms staples 130 into a suitable configuration for stapling tissue.

As seen in FIG. 6A, anvil channel 111 houses a translation rod 154 for longitudinally translating translatable tip 150 between the extended and retracted states upon a sliding of translation switch 32. A proximal end of translation rod 154 is operably connected to translation switch 32. A distal end of translation rod 154 is operably connected to translatable tip 150. Translation rod 154 may have multiple segments in embodiments of surgical stapling device 10 in which end effector 100 is releasably attached to elongated shaft 18. Cartridge channel 112 houses sled 122, as will be discussed in greater detail hereinbelow.

FIGS. 6A-8 illustrate transitioning of tool assembly 104. FIG. 6A illustrates a typical state of tool assembly 104 at a beginning of a surgical procedure. Tool assembly 104 is in a closed position and translatable tip 150 is retracted within anvil assembly 106 for tool assembly 104 to have a minimal configuration. A minimal configuration of tool assembly 104 assists in movement of tool assembly 104 through a surgical site. At the surgical site, rotation knob 14 and articulation knob 16 may be each rotated to a degree necessary to suitably position tool assembly 104 for grasping tissue.

Tool assembly 104 is transitioned from the open position to the closed position by partially translating drive member 120 distally through anvil channels 111, 112. In the grasping mode, drive member 120 cannot translate far enough distally to push sled 122 distally. In the firing mode, drive member 120 is translated far enough distally to push sled 122 distally. Tool assembly 104 is transitioned from the closed position to the open position by translating drive member 120 proximally.

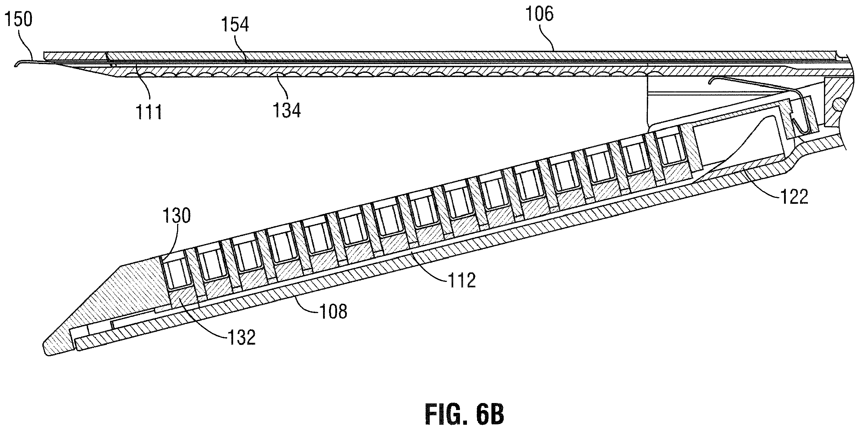

As seen in FIG. 6B, tool assembly 104 may be transitioned to the open position to receive tissue therebetween. To assist in manipulating tissue, translatable tip 150 may be extended from anvil channel 111. Once tissue is between anvil assembly 106 and cartridge assembly 108, tool assembly 104 can be transitioned to the closed position to grasp the tissue or fire staples therethrough. Translatable tip 150 may be retracted or may remain extended when tool assembly 104 is in the closed position.

As seen in FIGS. 7-8, staples can be fired when tool assembly 104 is in the closed position. First, surgical stapling device 10 is switched from the grasping mode to the firing mode by sliding actuator button 28 distally. Movable handle 22 is repeatedly pulled to the first position and released to the second position, each time incrementally advancing drive member 120 distally through channels 111, 112. Drive member 120 pushes sled 122 distally through cartridge assembly 108. Sled 122 sequentially engages pushers 132, which push staples 130 into anvil pockets 134. At any time surgical stapling device 10 is in the grasping mode, such as when stapling is complete, retraction handles 26 may be pulled proximally to return surgical stapling device 10 to the grasping mode. Then surgical stapling device 10 may be removed from the surgical site. To assist in removal, translatable tip 150 may be retracted within anvil channel 111 and tool assembly may be transitioned to the closed position to for a minimal configuration of tool assembly 104.

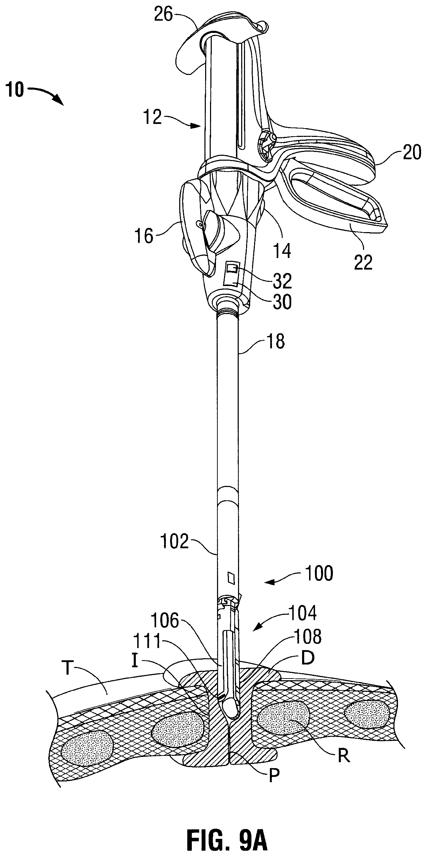

FIGS. 9A-9C illustrate a method of use of surgical stapling device 10. Although the method is described with respect to a thoracic procedure, it should be understood that substantially similar methods may also be used for endoscopic, laparoscopic, and other minimally invasive procedures. As seen in FIG. 9A, an incision I is created in a tissue T in an intercostal space between ribs R. An access device D is inserted through incision I to facilitate creating a substantially fluid-tight seal about incision I. Access device D may be formed of a compliant foam or plastic that conforms to tissue T. Alternatively, access device D may be formed of a substantially firm material to provide a force that spread ribs R apart to create a larger working area for the thoracic procedure. Access device could also be configured to stretch a flexible membrane to stretch the incision. Access device D has at least one port P extending therethrough for receipt of surgical stapling device 10 therethrough. During insertion of surgical stapling device 10 through portion P, surgical access device is in the grasping mode. Movable handle 22 is pulled to the first position to close tool assembly 104. Switch 32 is slid proximally to retract tip 150.

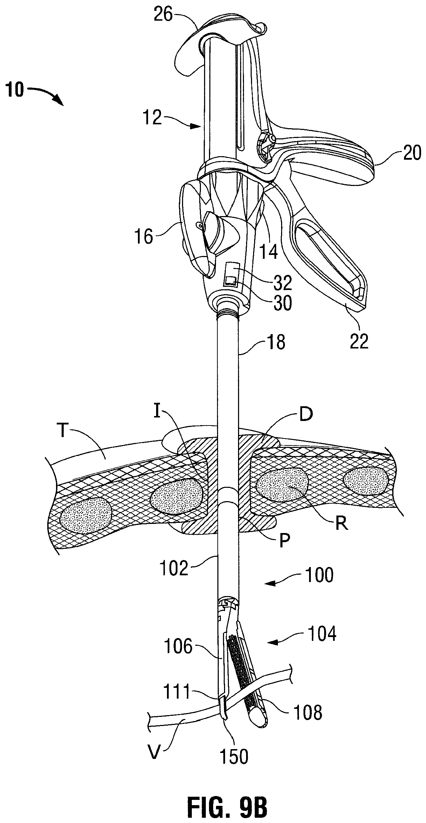

As seen in FIG. 9B, surgical access device 10 is inserted through port P. End effector 100 passes completely through port P. Handle assembly 12 remains proximal to port P. A portion of elongated member 18 is within port P, and material defining port P forms a substantially fluid-tight seal with the portion of elongated member 18 therein. Sliding switch 32 distally extends tip 150 to manipulate a vessel V underneath tissue T. Movable handle 22 may be pulled and released as necessary for tool assembly 104 to grasp, manipulate, and maneuver through tissue. Rotating actuator handle 16 and rotation knob 14 may assist in maneuvering end effector 100 through tissue.

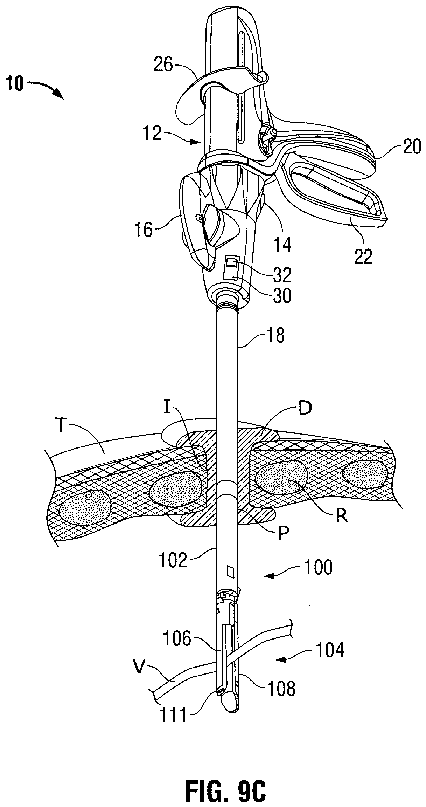

As seen in FIG. 9C, once vessel V has been grasped within tool assembly 104, staples 130 may be fired through vessel V. Actuation button 28 is slid distally to switch surgical device 10 to the firing mode, as seen in FIG. 3D. Movable handle 22 is repeatedly pulled and released to incrementally fire staples 130. Each pulling of movable handle 22 incrementally moves retraction handles 26 distally. When firing is complete, retraction handles 26 are pulled proximally to return surgical stapling device 10 to the grasping mode and release vessel V from tool assembly 104. Surgical stapling device 10 may then be removed from the surgical site by reversing the insertion steps described hereinabove.

FIG. 10 illustrates another embodiment of an anvil assembly of a surgical stapling device according to the present disclosure and is designated as 206. Anvil assembly 206 may replace or be used instead of anvil assembly 106. Anvil assembly 206 is similar to anvil assembly 106 and thus will only be discussed in detail herein to the extent necessary to identify differences in construction and operation thereof. Anvil assembly 206 includes a pneumatic tip 250. Pneumatic tip 250 is compliant. Pneumatic tip 250 has a rolled configuration in a retracted state and an unrolled configuration in an extended state. Pneumatic tip 250 is connected to a distal end of translation rod 154. Pneumatic tip 250 and translation rod 154 are hollow and adapted for receipt of a pressurized fluid, such as pressurized CO₂, therethrough. A proximal end of translation rod 154 is in fluid communication with any appropriate pneumatic supply, such as a pump or a compressed gas cartridge, for extending and retracting pneumatic tip 250. Pneumatic tip 250 may be biased to the retracted state, such that fluid pressure extends pneumatic tip 250 and removal of fluid pressure retracts pneumatic tip 250.

FIG. 11 illustrates an embodiment of a tool assembly of a surgical stapling device not according to the present invention and is generally designated as 304. Tool assembly 304 may replace or be used instead of tool assembly 104. Tool assembly 304 is similar to tool assembly 104 and thus will only be discussed in detail herein to the extent necessary to identify differences in construction and operation thereof. Tool assembly 304 includes an anvil assembly 306 and a cartridge assembly 308 that are pivotably movable with respect to each other between an open position and a closed position. Anvil assembly 306 has a kinked tip 350 for manipulating tissue. When tool assembly 304 is in the closed position, kinked tip 350 and a distal end of cartridge assembly 308 are substantially parallel and define a tissue engagement plane therebetween. Cartridge assembly 308 is longitudinally translatable between a retracted state and an extended state upon a sliding of translation switch 32. As anvil assembly 306 remains in place as cartridge assembly is retracted, retracting cartridge 308 widens the engagement plane, thus allowing kinked tip 350 to more easily manipulate larger tissue. In an alternate embodiment, anvil assembly 306 may be longitudinally translatable between a retracted state and an extended state as cartridge assembly 308 remains stationary. Embodiments are also conceived in which a distal tip of cartridge assembly 308 is distal to a distal tip of anvil assembly 306, in which case cartridge assembly 308 may be extendable or anvil assembly 306 may be retractable to widen the engagement plane.

FIG. 12 illustrates another embodiment of an end effector of a surgical stapling device not according to the present invention and is generally designated as 400. End effector 400 may replace or be used instead of end effector 100. End effector 400 is similar to end effector 100 and thus will only be discussed in detail herein to the extent necessary to identify differences in construction and operation thereof. End effector 400 includes a body portion 402 and a tool assembly 404. Tool assembly 404 includes an anvil assembly 406 and cartridge assembly 408 that are pivotably movable with respect to each other. Body portion 402 includes an extendable portion 402a that is extendable and retractable along the longitudinal axis defined by elongated member 18 to simultaneously extend or retract both anvil assembly 406 and cartridge assembly 408, thereby assisting the user in manipulating tissue. Extension and retraction of extendable portion 402a are actuated by sliding translation switch 32 distally and proximally, respectively.

Anvil assembly 406 has an LED tip 460 for use as a visual aid. Alternatively, cartridge assembly 408 could have an LED tip. LED tip 460 may be curved and/or tapered to assist in manipulating tissue. In another embodiment, at least one of anvil assembly 406 and cartridge assembly 408 has a sensor tip. The sensor tip may be used to sense a tissue condition. The sensor tip may be in wired or wireless communication with a processor for processing sensed information about the tissue condition.

Anvil assembly 406 includes a distal end of a tube 470. Tube 470 may serve many functions, such as evacuation of material within the surgical site, irrigation of the surgical site, and absorption of material from the surgical site. Tube 470 extends proximally from anvil assembly 406 through end effector 400 and further through elongated member 18. A proximal end of tube 470 may be operably connected to a mechanism, such as a vacuum source or an irrigation source, for fluid communication with the surgical site.

It will be understood that various modifications may be made to the embodiments disclosed herein. For example, it is envisioned that the surgical stapling device disclosed may be used in association with other surgical devices, e.g., clip appliers, dissectors, electrosurgical sealing devices, etc. Further, the device may also include tool assemblies other than staplers or those devices which eject a fastener, e.g., sealing devices (electrosurgical and non-electrosurgical), etc. Therefore, the above description should not be construed as limiting, but merely as exemplifications of preferred embodiments. Those skilled in the art will envision other modifications within the scope of the claims appended hereto.

## Claims

1. A surgical device comprising:
a handle assembly (12);
an elongated member (18) extending distally from the handle assembly, the elongated member defining a longitudinal axis;
a tool assembly (104) mounted to a distal end of the elongated member, the tool assembly having a cartridge assembly (108) having a plurality of staples (130) supported therein and an anvil assembly (106), at least one of the anvil assembly and the cartridge assembly being pivotably movable in relation to each other between open and closed positions,
wherein at least one of the cartridge assembly (108) and the anvil assembly (106) includes a tongue (150) configured to transition said at least one of the cartridge assembly and the anvil assembly between an extended state and a retracted state,
**characterized in that** the tongue is configured to transition out from and into said at least one of the cartridge assembly and the anvil assembly,
and the surgical device further comprises a transitioning mechanism configured to transition at least one of the anvil assembly (106) and the cartridge assembly (108) between the extended and retracted states wherein the transitioning mechanism includes a rod (154) for transitioning the tongue (150).

2. The surgical device according to claim 1, wherein the transitioning mechanism is configured to pneumatically transition the tongue (150).

3. The surgical device according to claim 1 or 2, wherein the tongue (150) has an arcuate configuration.

4. The surgical device according to claim 1, wherein the tongue (150) is composed of shape memory material and is configured to return to a shape memorized position in the extended state.

5. The surgical device according to claim 1, wherein the cartridge assembly (108) and the anvil assembly (106) are configured to move between the open and closed positions in response to actuation of a movable handle (22) of the handle assembly (12).

6. The surgical device according to claim 1, wherein the cartridge assembly (108) is configured to fire the plurality of staples (130) in response to actuation of a movable handle (22) of the handle assembly (12).

7. The surgical device according to claim 1, further including a mode selection mechanism configured to alternate the surgical device between a first mode of operation and a second mode of operation, the mode selection mechanism including a switch (28) for selecting between the first mode of operation, in which the cartridge assembly (108) and anvil assembly (106) are movable back and forth between the open and closed positions, and the second mode of operation, in which the cartridge assembly (108) and anvil assembly (106) are locked in the closed position for clamping tissue therebetween and firing the plurality of staples (130).

## Patentansprüche

1. Eine chirurgische Vorrichtung, aufweisend:
eine Handgriffanordnung (12);
ein längliches Element (18), das sich distal von der Handgriffanordnung erstreckt, wobei das längliche Element eine Längsachse definiert;
eine Werkzeuganordnung (104), die an einem distalen Ende von dem länglichen Element befestigt ist, wobei die Werkzeuganordnung eine Kartuschenanordnung (108) mit einer Vielzahl von darin abgestützten Klammern (130) und eine Ambossanordnung (106) hat, wobei wenigstens eine von der Ambossanordnung und der Kartuschenanordnung in Bezug aufeinander zwischen geöffneten und geschlossenen Positionen schwenkbar bewegt werden kann,
wobei wenigstens eine von der Kartuschenanordnung (108) und der Ambossanordnung (106) eine Zunge (150) umfasst, die dazu konfiguriert ist, für einen Übergang von der besagten wenigstens einen von der Kartuschenanordnung und der Ambossanordnung zwischen einem ausgefahrenen Zustand und einem eingefahrenen Zustand zu sorgen,
**dadurch gekennzeichnet, dass** die Zunge dazu konfiguriert ist, aus der besagten wenigstens einen von der Kartuschenanordnung und der Ambossanordnung heraus und in diese hinein überzugehen,
und die chirurgische Vorrichtung weiter einen Übergangsmechanismus aufweist, der dazu konfiguriert ist, für einen Übergang von wenigstens einer von der Ambossanordnung (106) und der Kartuschenanordnung (108) zwischen den ausgefahrenen und eingefahrenen Zuständen zu sorgen, und wobei der Übergangsmechanismus einen Stab (154) umfasst, um für einen Übergang der Zunge (150) zu sorgen.

2. Die chirurgische Vorrichtung nach Anspruch 1, wobei der Übergangsmechanismus dazu konfiguriert ist, für einen pneumatischen Übergang der Zunge (150) zu sorgen.

3. Die chirurgische Vorrichtung nach Anspruch 1 oder 2, wobei die Zunge (150) eine gekrümmte Konfiguration hat.

4. Die chirurgische Vorrichtung nach Anspruch 1, wobei die Zunge (150) aus einem Formgedächtnismaterial besteht und dazu konfiguriert ist, in dem ausgefahrenen Zustand in eine eingeprägte Formgedächtnisposition zurückzukehren.

5. Die chirurgische Vorrichtung nach Anspruch 1, wobei die Kartuschenanordnung (108) und die Ambossanordnung (106) dazu konfiguriert sind, sich als Reaktion auf eine Betätigung von einem beweglichen Handgriff (22) von der Handgriffanordnung (12) zwischen den geöffneten und geschlossenen Positionen zu bewegen.

6. Die chirurgische Vorrichtung nach Anspruch 1, wobei die Kartuschenanordnung (108) dazu konfiguriert ist, die Vielzahl von Klammern (130) als Reaktion auf eine Betätigung von einem beweglichen Handgriff (22) von der Handgriffanordnung (12) abzufeuern.

7. Die chirurgische Vorrichtung nach Anspruch 1, weiter einen Modusauswahlmechanismus umfassend, der dazu konfiguriert ist, die chirurgische Vorrichtung zwischen einem ersten Betriebsmodus und einem zweiten Betriebsmodus zu wechseln, wobei der Modusauswahlmechanismus einen Schalter (28) umfasst, um zwischen dem ersten Betriebsmodus, in welchem die Kartuschenanordnung (108) und Ambossanordnung (106) zwischen den geöffneten und geschlossenen Positionen zurück und vorwärts bewegt werden können, und dem zweiten Betriebsmodus auszuwählen, in welchem die Kartuschenanordnung (108) und Ambossanordnung (106) in der geschlossenen Position verriegelt sind, um Gewebe dazwischen einzuklemmen und die Vielzahl von Klammern (130) abzufeuern.

## Revendications

1. Dispositif chirurgical comprenant :
un ensemble poignée (12) ;
un élément allongé (18) s'étendant de façon distale à partir de l'ensemble poignée, l'élément allongé définissant un axe longitudinal ;
un ensemble outil (104) monté sur une extrémité distale de l'élément allongé, l'ensemble outil ayant un ensemble cartouche (108) ayant une pluralité d'agrafes (130) supportées en son sein et un ensemble enclume (106), au moins un de l'ensemble enclume et de l'ensemble cartouche étant mobile de manière pivotante par rapport à l'autre entre des positions ouverte et fermée,
dans lequel au moins un de l'ensemble cartouche (108) et de l'ensemble enclume (106) inclut une languette (150) configurée pour faire subir une transition audit au moins un de l'ensemble cartouche et de l'ensemble enclume entre un état étendu et un état rétracté,
**caractérisé en ce que** la languette est configurée pour subir une transition hors de et dans ledit au moins un de l'ensemble cartouche et de l'ensemble enclume,
et le dispositif chirurgical comprend en outre un mécanisme de transition configuré pour faire subir une transition à au moins un de l'ensemble enclume (106) et de l'ensemble cartouche (108) entre les états étendu et rétracté dans lequel le mécanisme de transition inclut une tige (154) pour faire subir une transition à la languette (150).

2. Dispositif chirurgical selon la revendication 1, dans lequel le mécanisme de transition est configuré pour faire subir une transition pneumatique à la languette (150).

3. Dispositif chirurgical selon la revendication 1 ou 2, dans lequel la languette (150) a une configuration curviligne.

4. Dispositif chirurgical selon la revendication 1, dans lequel la languette (150) est composée de matière à mémoire de forme et est configurée pour revenir à une position à forme mémorisée dans l'état étendu.

5. Dispositif chirurgical selon la revendication 1, dans lequel l'ensemble cartouche (108) et l'ensemble enclume (106) sont configurés pour se déplacer entre les positions ouverte et fermée en réponse à l'actionnement d'une poignée mobile (22) de l'ensemble poignée (12).

6. Dispositif chirurgical selon la revendication 1, dans lequel l'ensemble cartouche (108) est configuré pour tirer la pluralité d'agrafes (130) en réponse à l'actionnement d'une poignée mobile (22) de l'ensemble poignée (12).

7. Dispositif chirurgical selon la revendication 1, incluant en outre un mécanisme de sélection de mode configuré pour alterner le dispositif chirurgical entre un premier mode de fonctionnement et un second mode de fonctionnement, le mécanisme de sélection de mode incluant un commutateur (28) pour sélectionner entre le premier mode de fonctionnement, dans lequel l'ensemble cartouche (108) et l'ensemble enclume (106) sont mobiles dans les deux sens entre les positions ouverte et fermée, et le second mode de fonctionnement, dans lequel l'ensemble cartouche (108) et l'ensemble enclume (106) sont verrouillés dans la position fermée pour serrer le tissu entre ces derniers et tirer la pluralité d'agrafes (130).
